(19) Europäisches Patentamt
European Patent Office
Office européen des brevets

(11) Veröffentlichungsnummer: **0 161 220 B1**

(12) **EUROPÄISCHE PATENTSCHRIFT**

(45) Veröffentlichungstag der Patentschrift: **14.08.91**

(51) Int. Cl.$^5$: **C07D 277/74**, C07D 235/28

(21) Anmeldenummer: **85810210.6**

(22) Anmeldetag: **06.05.85**

Die Akte enthält technische Angaben, die nach dem Eingang der Anmeldung eingereicht wurden und die nicht in dieser Patentschrift enthalten sind.

(54) **Verfahren zur Herstellung von beta-(Benzthiazolylthio)- und beta-(Benzimidazolylthio)-carbonsäurederivaten.**

(30) Priorität: **11.05.84 GB 8412065**

(43) Veröffentlichungstag der Anmeldung:
**13.11.85 Patentblatt 85/46**

(45) Bekanntmachung des Hinweises auf die Patenterteilung:
**14.08.91 Patentblatt 91/33**

(84) Benannte Vertragsstaaten:
**AT DE FR GB IT**

(56) Entgegenhaltungen:
**EP-A- 0 093 908**
**EP-A- 0 126 030**
**EP-A- 0 129 506**
**US-A- 2 725 364**
**US-A- 3 068 239**
**US-A- 3 136 689**
**US-A- 3 161 495**

**J. Org. Chem. (1962) 27, 3140-3146**

**J. March, Advanced Organic Chemistry (1984) 2nd edition, chap. 15, p. 703**

**J. Org. Chem. (1971) 36, 636-641**

**Organic Magnetic Resonance (1978) 11, 617-627**

**H. J. Hediger, Infrarotspektroskopie, in: Methoden der Analyse in der Chemie (1971) Bd. 11, S. 141**

(73) Patentinhaber: **CIBA-GEIGY AG**
**Klybeckstrasse 141**
**CH-4002 Basel(CH)**

(72) Erfinder: **Baumann, Marcus, Dr.**
**Hammerstrasse 82**
**CH-4057 Basel(CH)**
Erfinder: **Bosshard, Hans**
**Hohe Winde-Strasse 23**
**CH-4059 Basel(CH)**
Erfinder: **Greuter, Hans, Dr.**
**Bleumatthöhe 10**
**CH-5264 Gipf-Oberfrick(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 161 220 B1

## Beschreibung

Die vorliegende Erfindung betrifft ein Verfahren zur Herstellung von Estern, Amiden, Imiden und Anhydriden von aliphatischen oder cycloaliphatischen Carbonsäuren, welche in ß-Stellung durch einen heterocyclischen Mercaptorest substituiert sind und das dadurch gekennzeichnet ist, dass man ein a,ß-ungesättiges Carbonsäurederivat in stark saurem Medium mit einem heterocyclischen Mercaptan reagieren lässt.

Die Addition von Mercaptanen an a,ß-ungesättigte Säuren und deren Derivate ist prinzipiell bekannt. Sie wird jedoch üblicherweise in einem basischen Medium oder unter Verwendung basischer Katalysatoren ausgeführt. Man nimmt an, dass dabei der erste Schritt in einer Anlagerung des Mercaptid-Anions an das ß-C-Atom der Carbonsäure besteht. F.B. Ziently et al. (J. Org. Chem. 27 (1962, 3140) beschrieben die Addition von verschiedenen Thiolen an Maleinsäureanhydrid unter basischer Katalyse. Diese Autoren bemerken, dass die Addition bei radikalischer Katalyse nur mässige Ausbeuten ergibt und dass Lewis-Säuren keine katalysierende Wirkung haben.

Im US-Patent 2,725,364 wird im Beispiel 2 gezeigt, dass sich Dibutylmaleinat unter basischer Katalyse an 2-Mercaptobenzthiazol anlagern lässt. Wie eigene Untersuchungen zeigten entsteht dabei in mässiger Ausbeute ein Gemisch der N- und S-Addukte.

Im US-Patent 3,068,239 wird die basenkatalysierte Anlagerung von Acetylencarbonsäureestern an 2-Mercaptobenzthiazol, -imidazol und -oxazol beschrieben. Dabei bildet sich im Falle des Benzimidazols ebenfalls ein Gemisch der isomeren S- und N-Addukte.

Es ist weiter bekannt aus J. Org. Chem. 36 (1971), 636, dass bei der Michael-Reaktion von Acrylnitril, Vinylketonen, Vinylpyridin oder Vinylsulfon mit Mercaptobenzthiazol unter basischer Katalyse eine N-Addition eintritt und sich die entsprechenden Benzthiazolinthion-Derivate bilden. Ueberraschenderweise wurde nunmehr gefunden, dass in stark saurem Medium die Addition von a,ß-ungesättigten Carbonsäureestern, -amiden, und imiden an 2-Mercaptobenzthiazol unter S-Substitution verläuft und die entsprechenden ß-[Benzthiazolyl-2-mercapto]-carbonsäurederivate in hoher Ausbeute und Reinheit entstehen.

Gegenstand der Erfindung ist daher ein Verfahren zur Herstellung von Verbindungen der Formel I,

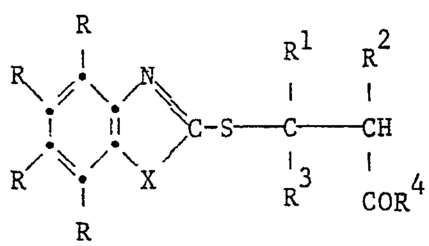

I

worin X Schwefel oder NH bedeutet, jedes R unabhängig von den anderen Wasserstoff, $C_1$-$C_{12}$-Alkyl, $C_1$-$C_4$-Halogenalkyl, $C_1$-$C_4$-Alkoxy, $C_1$-$C_4$-Alkylthio, $C_1$-$C_{12}$-Alkylsulfonyl, Phenyl, $C_7$-$C_{12}$-Alkylphenyl, $C_7$-$C_{12}$-Phenylalkyl, $C_5$-$C_8$-Cycloalkyl, Halogen, -$NO_2$, -CN, -COOH, -COO-$C_1$-$C_4$-Alkyl, -OH oder eine Amino- oder Carbamoylgruppe bedeutet, und $R^1$, $R^2$ und $R^3$ unabhängig voneinander Wasserstoff, $C_1$-$C_{12}$-Alkyl, $C_1$-$C_4$-Halogenalkyl, $C_1$-$C_4$-Hydroxyalkyl, $C_2$-$C_{10}$-Alkoxyalkyl, Carboxyl oder $C_2$-$C_{12}$-Carboxyalkyl, Carbamoyl oder $C_2$-$C_{16}$-Carbamoylalkyl oder unsubstituiertes oder durch Halogen, Nitro, Hydroxy, oder Carboxy substituiertes Phenyl oder Benzyl bedeuten oder $R^1$ und $R^2$ zusammen eine direkte Bindung bedeuten,

$R^4$ eine unsubstituierte oder durch $C_1$-$C_{12}$-Alkyl, $C_6$-$C_{16}$-Aryl, $C_7$-$C_{16}$-Aralkyl oder $C_3$-$C_8$-Cycloalkyl substituierte Aminogruppe, eine $C_1$-$C_{12}$-Alkoxygruppe, eine $C_5$-$C_8$-Cycloalkoxygruppe, eine $C_6$-$C_{16}$-Aryloxygruppe oder eine $C_7$-$C_{16}$-Aralkoxygruppe bedeutet oder $R^2$ und $R^4$ zusammen eine Gruppe -$CH_2$-CO-$NR^5$-bilden, oder $R^3$ und $R^4$ zusammen eine Gruppe -CO-$NR^5$-bilden und

$R^5$ Wasserstoff, $C_1$-$C_{12}$-Alkyl, Cyclohexyl, $C_6$-$C_{16}$-Aryl oder $C_7$-$C_{16}$-Aralkyl bedeutet, durch Reaktion eines heterocyclischen Mercaptanes der Formel II

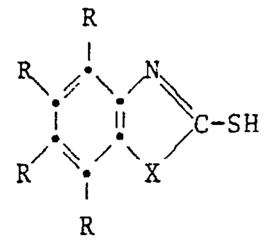

II

mit einem ungesättigten Carbonsäurederivat der Formel III

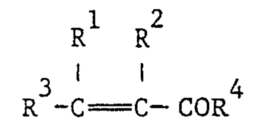

III

in stark saurem Medium.

Wenn in Formel I und II R Alkyl ist, so kann dies unverzweigtes oder verzweigtes Alkyl sein und ist vorzugsweise $C_1$-$C_{12}$-Alkyl wie z.B. Methyl, Ethyl, Propyl, Isopropyl, n-Butyl, Isobutyl, tert.-Butyl, Pentyl, Hexyl, Octyl, Nonyl, Decyl oder Dodecyl. R als Halogenalkyl ist vorzugsweise $C_1$-$C_4$-Halogenalkyl, wie z.B. Chlormethyl, Mono-, Di-, Trifluormethyl, Trichlormethyl oder 2-Chlorethyl. R als Alkoxy oder Alkylthio hat vorzugsweise 1-4 C-Atome und kann z.B. Methoxy, Ethoxy, Isopropoxy, Methylthio, Propylthio oder tert.-Butylthio sein. R als Alkylsulfonyl ist vorzugsweise $C_1$-$C_{12}$-Alkylsulfonyl und kann z.B. Methylsulfonyl, tert.-Butylsulfonyl, n-Octylsulfonyl oder n-Dodecylsulfonyl sein. R als Cycloalkyl enthält vorzugsweise 5-8 C-Atome. Beispiele hierfür sind Cyclopentyl, Cyclohexyl oder Cyclooctyl. R als Alkylphenyl oder Phenylalkyl hat vorzugsweise 7 - 12 C-Atome und kann z.B. Tolyl, Xylyl, Ethylphenyl, tert.-Butylphenyl, Benzyl, 1- oder 2-Phenylethyl oder $\alpha,\alpha$-Dimethylbenzyl sein. R als -COOAlkyl ist z.B. Methoxycarbonyl, Ethoxycarbonyl oder Butoxycarbonyl.

Vorzugsweise verwendet man eine Verbindung der Formel II, worin mindestens zwei der Substituenten R Wasserstoff sind, insbesondere Verbindungen der Formel II, worin ein R Wasserstoff, $C_1$-$C_4$-Alkyl, $C_1$-$C_4$-Alkoxy, Halogen, -COOH oder eine Aminogruppe ist und die anderen drei R Wasserstoff sind.

Bevorzugt verwendet man Verbindungen der Formel II, worin X Schwefel ist und erhält dabei die entsprechenden ß-(Benzthiazol-2-ylthio)carbonsäurederivate.

Wenn in Formel I und III die Substituenten $R^1$, $R^2$ oder $R^3$ Alkyl bedeuten, so können sie unverzweigtes oder verzweigtes Alkyl bedeuten. Beispiele hierfür sind
Methyl, Ethyl, n-Propyl, Isopropyl, n-Butyl, sec.-Butyl, Isobutyl, n-Pentyl, Isopentyl, Hexyl, Heptyl, Octyl, Nonyl, Decyl oder Dodecyl. Sind $R^1$, $R^2$ oder $R^3$ Halogenalkyl oder Hydroxyalkyl, so haben sie 1-4 C-Atome. Beispiele hierfür sind Hydroxymethyl, 1- oder 2-Hydroxyethyl, 1-, 2- oder 3-Hydroxypropyl, 3-Hydroxybutyl, Chlormethyl, Mono-, Di- oder Trifluormethyl, Brommethyl, 2-Chlorethyl, 3-Chlorpropyl oder 2-Chlorbutyl.

$R^1$, $R^2$ und $R^3$ als Alkoxyalkyl sind
z.B. Methoxymethyl, 1- oder 2-Methoxyethyl, Ethoxymethyl, 2-Butoxyethyl oder Octyloxymethyl. $R^1$ , $R^2$ und $R^3$ als Carboxyalkyl sind
z.B. Carboxymethyl, 1- oder 2-Carboxyethyl, 2- oder 3-Carboxypropyl, 1- oder 4-Carboxybutyl oder 6-Carboxyhexyl. Wenn $R^1$, $R^2$ oder $R^3$ Carbamoylalkyl bedeutet, so hat darin die Alkylgruppe 2-16 C-Atome. Das N-Atom einer solchen Carbamoylalkylgruppe kann durch aliphatische oder aromatische Reste mono-oder disubstituiert sein, wobei diese Substituenten 1-12 C-Atome haben können. Vorzugsweise ist das N-Atom einer solchen Carbamoylgruppe unsubstituiert.

Bevorzugt sind $R^1$, $R^2$ und $R^3$ Wasserstoff, $C_1$-$C_8$-Alkyl, Carboxyl, $C_2$-$C_8$-Carboxylalkyl, Carbamoyl, $C_2$-$C_{16}$-Carbamoylalkyl oder Phenyl. Besonders bevorzugt sind mindestens zwei der Substituenten $R^1$, $R^2$, $R^3$ Wasserstoff.

Vorzugsweise enthält $R^4$ als Aminogruppe bis zu 20 C-Atome.

$R^4$ als Alkoxygruppe hat 1-12 C-Atome, insbesondere 1-6 C-Atome. Beispiele hierfür sind Methoxy, Ethoxy, Propyloxy, Butyloxy, Pentyloxy, Hexyloxy, Octyloxy oder Dodecyloxy. $R^4$ als Cycloalkoxy ist vorzugsweise $C_5$-$C_8$-Cycloalkoxy, wie z.B. Cyclopentyloxy oder Cyclohexyloxy. $R^4$ als Aryloxy ist Z.B. Phenoxy, Methylphenoxy oder Chlorphenoxy. $R^4$ als Aralkoxy ist z.B. Benzyloxy oder Methylbenzyloxy.

$R^4$ ist vorzugsweise $C_1$-$C_6$-Alkoxy, Cyclohexyloxy, Cyclopentyloxy, Phenyloxy, Benzyloxy, $-NH_2$ oder

$-NR^6R^7$, worin $R^6$ $C_1$-$C_4$-Alkyl ist und $R^7$ Wasserstoff oder $C_1$-$C_4$-Alkyl ist.

$R^5$ als Alkyl hat 1-12, insbesondere 1-4 C-Atome. Beispiele hierfür sind Methyl, Ethyl, n-Propyl, i-Propyl, Butyl, n-Hexyl, 2-Ethylbutyl, n-Octyl, 2-Ethylhexyl oder n-Dodecyl. $R^5$ als Aryl ist z.B. Phenyl, Tolyl oder 4-Butylphenyl.

$R^5$ als Aralkyl ist vorzugsweise Benzyl.

$R^5$ ist vorzugsweise Wasserstoff, $C_1$-$C_4$-Alkyl, Cyclohexyl, Phenyl oder Benzyl.

Die Mercaptane der Formel II sind bekannte Verbindungen oder können in Analogie zu diesen hergestellt werden. Beispiele für erfindungsgemäss verwendbare Verbindungen der Formel II sind: 2-Mercaptobenzthiazol, 5-Methyl-2-mercaptobenzthiazol, 4-Isopropyl-2-mercaptobenzthiazol, 7-t-Butyl-2-mercapto-benzthiazol, 6-Cyclohexyl-2-mercapto-benzthiazol, 7-Benzyl-2-mercapto-benzthiazol, 5-Trifluormethyl-2-mercapto-benzthiazol, 6-Methoxy-2-mercapto-benzthiazol, 7-Ethoxy-2-mercaptobenzthiazol, 4-Methylthio-2-mercapto-benzthiazol, 6-Methylsulfonyl-2-mercaptobenzthiazol, 4-Fluor-2-mercapto-benzthiazol,5-Chlor-2-mercapto-benzthiazol, 7-Brom-2-mercapto-benzthiazol, 6-Chlor-2-mercapto-benzthiazol, 4-Phenyl-2-mercapto-benzthiazol, 6-Nitro-2-mercapto-benzthiazol, 5-Cyano-2-mercapto-benzthiazol, 5-Carboxy-2-mercapto-benzthiazol, 5-Methoxycarbonyl-2-mercapto-benzthiazol, 7-Hydroxy-2-mercapto-benzthiazol, 6-Amino-2-mercapto-benzthiazol, 5-Carbamoyl-2-mercapto-benzthiazol, 5-Chlor-6-n-butyl-2-mercapto-benzthiazol, 5-Nitro-6-n-propyl-2-mercapto-benzthiazol, 5-Brom-6-n-propoxy-2-mercapto-benzthiazol, 4,5,6-Triethyl-2-mercapto-benzthiazol, 4,5,6,7-Tetramethyl-2-mercapto-benzthiazol, 4-Methoxy-6-hydroxy-2-mercapto-benzthiazol, 4,5-Dimethyl-7-propoxy-2-mercapto-benzthiazol, 2-Mercaptobenzimidazol, 6-Methyl-2-mercapto-benzimidazol, 4-Isopropyl-2-mercapto-benzimidazol, 5-n-Hexyl-2-mercapto-benzimidazol, 6-(1,1,3,3-Tetramethylbutyl)-2-mercapto-benzimidazol, 7-Benzyl-2-mercapto-benzimidazol, 6-Ethoxy-2-mercapto-benzimidazol, 6-Isopropoxy-2-mercapto-benzimidazol, 4-Fluor-2-mercapto-benzimidazol, 5-Chlor-2-mercapto-benzimidazol, 5-Chlor-2-mercapto-benzimidazol, 5-Cyano-2-mercapto-benzimidazol, 4-Phenyl-2-mercapto-benzimidazol, 6-Nitro-2-mercapto-benzimidazol, 5-Carboxy-2-Mercapto-benzimidazol, 5-Butoxycarbonyl-2-mercapto-benzimidazol, 7-Hydroxy-2-mercapto-benzimidazol, 6-Amino-2-mercapto-benzimidazol, 4-Brom-5-n-hexyl-2-mercapto-benzmidazol, 5-Nitro-6-n-propy-2-mercapto-benzimidazol, 4,5,6-Triethyl-2-mercapto-benzimidazol oder 4,5-Dimethyl-7-propoxy-2-mercapto-benzimidazol.

Die Carbonsäurederivate der Formel III können Ester oder partielle Ester, Amide, partielle Amide oder Imide sein. Beispiele sind die entsprechenden Derivate von

Acrylsäure, Methacrylsäure, Crotonsäure, 2,3- oder 3,3-Dimethylacrylsäure, Propiolsäure, Phenylpropiolsäure, Maleinsäure, Fumarsäure, Acetylendicarbonsäure, Itaconsäure, Cyclohexen-1,2-dicarbonsäure, 3-Methylcyclohexen-1,2-dicarbonsäure, Ethylentetracarbonsäure, Mesaconsäure, Glutaconsäure, Aconitsäure, Citraconsäure, $\alpha$-Methylenglutarsäure, $\alpha$-Methylenadipinsäure, $\alpha$-Ethyliden-adipinsäure, Propylen-1,3-dicarbonsäure, 1-Buten-1,4-dicarbonsäure, 1-Buten-2,3,4-tricarbonsäure, 2-Pentensäure, 2-Hexensäure, 2-Octensäure, 2-Decensäure, 2-Undecensäure, 2-Dodecensäure, 2-Octadecensäure, Zimtsäure, $\alpha$-Phenylacrylsäure, $\alpha$-Phenylcrotonsäure, $\beta$-Benzylacrylsäure, Benzylidenmalonsäure, $\alpha$-Methylzimtsäure, 4-Chlorzimtsäure oder 3-Nitrozimtsäure.

Bevorzugt verwendet man ungesättigte Dicarbonsäurederivate der Formel III, worin $R^3$ Carboxyl oder Carbamoyl ist oder $R^2$ Carboxymethyl oder Carbamoylmethyl ist. Besonders bevorzugt sind die entsprechenden Maleinsäurederivate.

Die Reaktion von II mit III wird in stark saurem Medium ausgeführt. Das Reaktionsmedium kann z.B. eine wässrige Lösung einer Mineralsäure sein wie z.B. von $H_2SO_4$, $H_3PO_4$, HCl, HBr, $HBF_4$, $HClO_4$, $H_2S_2O_7$ oder Polyphosphorsäure. Organische Säuren wie z.B. Ameisensäure, Trifluoressigsäure oder p-Toluolsulfonsäuren können in wässriger Lösung oder in organischer Lösung eingesetzt werden. Bestimmte Säuren können auch in unverdünnter Form als Reaktionsmedium dienen, z.B. Trifluoressigsäure, Ameisensäure oder Phosphorsäure.

Als Säuren können auch Lewis-Säuren verwendet werden, wie z.B. $AlCl_3$, $AlBr_3$, $BF_3$, $SbF_5$, $SbCl_5$ oder $SnCl_4$. In diesem Fall führt man die Reaktion in einem inerten Lösungsmittel aus, in dem die Lewis-Säure löslich ist, beispielsweise in Diethylether oder in halogenierten Kohlenwasserstoffen.

Wenn die Ausgangsmaterialien in der verwendeten wassrigen Säure nicht löslich sind, so kann man auch ein mit Wasser mischbares organisches Lösungsmittel zusetzen, beispielsweise Methanol, Ethanol, Ethylenglykolmonomethylether, Essigsäure, Propionsäure, Tetramethylensulfon (Sulfolan), Tetrahydrofuran, Dioxan, Aceton oder Dimethylsulfoxid. In diesem Fall arbeitet man also in einem sauren wässrigorganischen Medium.

Bevorzugt führt man die Reaktion in einer wässrigen oder wässrigorganischen Lösung einer starken Protonsäure aus, insbesondere in 60-90 %iger Schwefelsäure oder in 25-38 %iger Salzsäure.

Die Reaktionstemperatur kann im Bereich von -30 °C bis zum Siedepunkt des Reaktionsmediums liegen, vorzugsweise arbeitet man bei 0° bis 100 °C. Unter bestimmten Bedingungen kann es vorteilhaft

sein, die Reaktion unter Ueberdruck auszuführen, jedoch ist dies nicht notwendig.

Die Reaktionskomponenten werden im annähernden Molverhältnis 1:1 eingesetzt, wobei man das Carbonsäurederivat III in geringem Ueberschuss einsetzt, bis zu etwa 10 Mol-%. Man kann zuerst die eine Komponente im sauren Reaktionsmedium lösen oder dispergieren und dann die zweite Komponente zugeben. Oder man mischt zuerst die beiden Komponenten und trägt dieses Gemisch langsam in das saure Reaktionsmedium ein.

Die Isolierung der Produkte kann nach üblichen Methoden erfolgen. Beim Arbeiten in einer konzentrierten Mineralsäure ist es zweckmässig, nach Beendigung der Reaktion das Reaktionsgemisch mit Wasser zu verdünnen und einen Teil der Mineralsäure durch Zugabe einer Base wie NaOH oder $NaCO_3$ zu neutralisieren, wobei das Produkt nach dem Abkühlen meist ausfällt oder durch Extraktion isoliert werden kann.

Das rohe Produkt kann durch Umfällung aus einer wässrigen Base gereinigt werden. Im allgemeinen fallen gemäss dem erfindungsgemässen Verfahren die Produkte in hoher Reinheit aus, so dass oft eine weitere Reinigung unnötig ist.

In den älteren Anmeldungen EP-A 129,506 und 126,030 wird eine analoge Additionsreaktion von ungesättigten Carbonsäuren oder deren Anhydriden an Mercaptane der Formel II in stark saurem Medium beschrieben. Wie in den Parallelanmeldungen EP-A 161,219 und 161,222 beschrieben ist, können die Verbindungen der Formel I als Korrosionsinhibitoren für Ueberzugsmittel oder für wässrige Systeme verwendet werden. Die Erfindung wird durch die nachfolgenden Beispiele näher erläutert. Die Temperaturen werden darin in °C angegeben.

Beispiel 1: 16,8 g (0,1 Mol) fein pulverisiertes 2-Mercaptobenzthiazol werden in 120 ml 70 %iger wässeriger Schwefelsäure suspendiert. Dazu werden unter gutem Rühren innert 30 Minuten 25,3 g (0,105 Mol) Maleinsäuredibutylester bei einer Temperatur von 0-10° getropft. Anschliessend wird 5 Stunden bei 0-10° gerührt, auf Eiswasser gegossen und mit Essigester extrahiert. Nach dem Abdampfen des Essigesters werden 32 g Rohprodukt erhalten, welches zur Weiterreinigung mit Essigester:Hexan (1:3) über Kieselgel filtriert wird. Durch Eindampfen der Lösung erhält man den Benzthiazol-2-ylthio-bernsteinsäuredibutylester als gelbliches Oel, $n_D^{20}$ 1,5515.

Analyse: $C_{19}H_{25}NO_4S_2$ (395,5)

| | | | | |
|---|---|---|---|---|
| ber: | C 57,7 % | H 6,4 % | O 16,2 % | S 16,2 % |
| gef: | 57,6 % | 6,6 % | 16,5 % | 15,5 % |

In analoger Weise erhält man bei Verwendung von Maleinsäure-diethylester den Benzthiazol-2-ylthio-bernsteinsäurediethylester, $n_D^{20}$ 1,5765.

Analyse: $C_{15}H_{17}NO_4S_2$ (339,4)

| | | | | | |
|---|---|---|---|---|---|
| ber: | C 53,08 % | H 5,05 % | N 4,13 % | O 18,86 % | S 18,89 % |
| gef: | 53,2 % | 5,0 % | 4,1 % | 19,1 % | 18,7 % |

Beispiel 2: 16,8 g (0,1 Mol) fein pulverisiertes 2-Mercaptobenzthiazol werden in 150 ml 70 %iger Schwefelsäure suspendiert. Dazu werden unter gutem Rühren innert 30 Minuten 16,6 g (0,105 Mol) Itaconsäuredimethylester bei einer Temperatur von 0-10° getropft. Anschliessend wird 16 Stunden bei 0-10° gerührt, auf Eiswasser gegossen und mit Essigester extrahiert. Die organische Phase wird abgetrennt, getrocknet und eingedampft. Man erhält den 3-(Benzthiazol-2-ylthio)-propan-1,2-dicarbonsäuredimethylester, der nach Umkristallisation aus Cyclohexan bei 46-47° schmilzt.

Analyse: $C_{14}H_{15}NO_4S_2$ (325,4)

| | | | | |
|---|---|---|---|---|
| ber: | C 51,68 % | H 4,65 % | N 4,31 % | S 19,71 % |
| gef: | 51,8 % | 4,7 % | 4,2 % | 19,5 % |

Beispiel 3: 16,8 g fein pulverisiertes 2-Mercaptobenzthiazol werden in 130 ml 70 %iger Schwefelsäure suspendiert. Innerhalb von 1 Stunde werden unter gutem Rühren bei 0-10° 11,0 g Acrylsäure-ethylester zugetropft. Anschliessend wird bei 0-10° während 1 1/2 Stunden gerührt, auf Eiswasser gegossen und mit Essigester extrahiert. Nach dem Abdampfen des Essigesters werden 18,3 g roher 3-(Benzthiazol-2-yl-thio)-propionsäureethylester erhalten, $n_D^{20}$ 1,6120.

Analyse: $C_{12}H_{13}NO_2S_2$ (267,4)

| | C | H | N | O | S |
|---|---|---|---|---|---|
| ber: | 53,91 % | 4,90 % | 5,24 % | 11,97 % | 23,98 % |
| gef: | 53,6 % | 4,9 % | 5,3 % | 12,0 % | 24,0 % |

Beispiel 4: In 75 ml 70 %iger, wässeriger Schwefelsäure werden 16,8 g fein pulverisiertes 2-Mercaptobenzthiazol verrührt und innerhalb von einer Stunde bei 0-10° 12,0 g Crotonsäureethylester zugetropft. Man lässt 5 Stunden bei 0-10° ausreagieren. Anschliessend wird auf Eiswasser gegossen und mit Essigester extrahiert. Nach dem Abdampfen des Essigesters werden 26,1 g Rohprodukt erhalten, welches zur Weiterreinigung mit Essigester/Hexan 1:3 über Kieselgel filtriert wird. Auf diese Weise werden 17,1 g 3-(Benzthiazol-2-yl-thio)-buttersäure-ethylester erhalten,$n_D^{20}$ 1,5965.

Analyse: $C_{13}H_{15}NO_2S_2$ (281,4)

| | C | H | N | O | S |
|---|---|---|---|---|---|
| ber: | 55,49 % | 5,37 % | 4,98 % | 11,37 % | 22,79 % |
| gef: | 55,7 % | 5,4 % | 5,0 % | 11,4 % | 22,5 % |

Beispiel 5: Eine fein gepulverte Mischung von 16,8 g 2-Mercaptobenzthiazol und 7,5 g Acrylamid wird innert einer Stunde bei 45-50° in 100 ml 70 % $H_2SO_4$ eingetragen. Während 1 Stunde lässt man ausreagieren bei 45-50° und giesst die Lösung auf Eiswasser. Der Niederschlag wird abfiltriert, mit Wasser gewaschen und getrocknet. Man erhält 22,1 g Rohprodukt von 3-(Benzthiazol-2-yl)-propionsäureamid, das nach Umkristallisation aus Essigester bei 144-145° schmilzt.

Analyse: $C_{10}H_{10}N_2OS_2$ (238,5)

| | C | H | N | S |
|---|---|---|---|---|
| ber: | 50,40 % | 4,23 % | 11,76 % | 26,91 % |
| gef: | 50,2 % | 4,3 % | 11,6 % | 26,6 % |

Beispiel 6: Eine fein-gepulverte Mischung von 16,8 g 2-Mercaptobenzthiazol und 15,1 g Fumarsäuremonoethylester wird innerhalb 1 Stunde bei 0-5° unter Rühren in 150 ml 70%ige $H_2SO_4$ eingetragen. Nach weiteren 5 Stunden Rühren bei 0-10° wird das Reaktionsgemisch auf Eiswasser gegossen und dieses mit Ethylacetat extrahiert. Die organische Lösung wird eingedampft, das hinterbleibende Rohprodukt (22,8 g) wird wie in Beispiel 1 gereinigt. Man erhält ein Isomerengemisch der beiden möglichen (Benzthiazol-2-ylthio)-bernsteinsäuremonoethylester als viskose Masse.

Analyse: $C_{13}H_{13}NO_4S_2$

| | C | H | N | S |
|---|---|---|---|---|
| ber: | 50,4 % | 4,2 % | 4,5 % | 20,9 % |
| gef. | 50,2 % | 4,3 % | 4,6 % | 20,6 % |

Beispiel 7: 16,8 g (0,1 Mol) 2-Mercaptobenzthiazol werden in 100 ml 70 % $H_2SO_4$ suspendiert. Dazu werden innert 1 Stunde bei 0-5° unter Rühren 13,7 g (0,105 Mol) Maleinsäuremonomethylester getropft. Anschliessend wird 5 Stunden bei 10° verrührt, auf Eis gegossen und mit Essigester extrahiert. Nach dem Abdampfen des Lösungsmittels werden 29,8 g Rohprodukt erhalten, das aus einem Gemisch der beiden isomeren (Benzthiazol-2-ylthio)-bernsteinsäure-monomethylester besteht. Durch Chromatographie über Kieselgel und Umkristallisation aus Essigester erhält man das eine Isomere, das bei 87-89° schmilzt.

Analyse: $C_{12}H_{11}NO_4S_2$ (297,3)

| | | | | |
|---|---|---|---|---|
| ber: | C 48,5 % | H 3,7 % | N 4,7 % | S 21,6 % |
| gef: | 47,8 % | 3,7 % | 4,6 % | 21,0 % |

Beispiel 8: 16,8 g (0,1 Mol) 2-Mercaptobenzthiazol werden in 200 ml 7o % $H_2SO_4$ suspendiert. Dazu werden innert 1 Stunde bei 10° unter Rühren 15 g Acetylendicarbonsäure-dimethylester getropft. Anschliessend wird 16 Stunden bei 20-25° verrührt, auf Eis gegossen und mit Essigester extrahiert. Nach dem Abdampfen des Lösungsmittels erhält man 27 g Rohprodukt, das ein Gemisch der beiden isomeren (Benzthiazol-2-ylthio)-maleinsäure-monomethylester darstellt. Durch Chromatographie über Kieselgel und Umkristallisation aus Essigester erhält man daraus das eine Isomere, das bei 144° schmilzt.

Analyse: $C_{12}H_9NO_4S_2$

| | | | | |
|---|---|---|---|---|
| ber: | C 48,8 % | H 3,1 % | N 4,7 % | S 21,7 % |
| gef: | 48,8 % | 3,2 % | 4,5 % | 21,1 % |

Beispiel 9: 16,4 g (0,1 Mol) 2-Mercaptobenzthiazol werden in 160 ml 70 % $H_2SO_4$ suspendiert. Dazu werden innert 30 Minuten bei 5° unter Rühren 26 g (0,105 Mol) But-3-en-1,2-dicarbonsäure-diethylester-3-carbonsäuremethylester getropft. Anschliessend wird 16 Stunden bei 20-25° verrührt, auf Eis gegossen und mit Methylenchlorid extrahiert. Nach dem Abdampfen des Lösungsmittels werden 28 g Rohprodukt erhalten, welches durch Filtration über eine Kieselgelsäule mit Essigester/ Methanol (1:3) gereinigt wird. Auf diese Weise wird der 4-(Benzthiazol-2-yl-thio)-butan-3-carbonsäuremethylester-1,2-dicarbonsäureethylester als dickflüssiges Oel erhalten.

NMR (250 MHz, $CDCl_3$): 1,25 (6H); 2,7 (1H); 3,0 (1H); 3,45 (5H); 3,75 (4H); 4,2 (2H); 7,35 (2H); 7,8 (2H).

**Patentansprüche**

1. Verfahren zur Herstellung von Verbindungen der Formel I

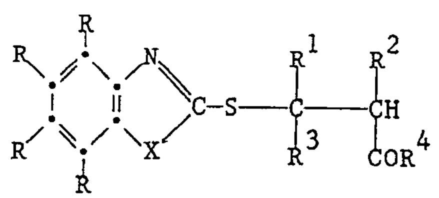

I,

worin X Schwefel oder NH bedeutet,
jedes R unabhängig von den anderen Wasserstoff, $C_1$-$C_{12}$-Alkyl, $C_1$-$C_4$-Halogenalkyl, $C_1$-$C_4$-Alkoxy, $C_1$-$C_4$-Alkylthio, $C_1$-$C_{12}$-Alkylsulfonyl, Phenyl, $C_7$-$C_{12}$-Alkylphenyl, $C_7$-$C_{12}$-Phenylalkyl, $C_5$-$C_8$-Cycloalkyl, Halogen, $-NO_2$, -CN, -COOH, -COO-$C_1$-$C_4$-Alkyl, -OH oder eine Amino- oder Carbamoylgruppe bedeutet, und $R^1$, $R^2$ und $R^3$ unabhängig voneinander Wasserstoff, $C_1$-$C_{12}$-Alkyl, $C_1$-$C_4$-Halogenalkyl, $C_1$-$C_4$-Hydroxyalkyl, $C_2$-$C_{10}$-Alkoxyalkyl, Carboxyl oder $C_2$-$C_{12}$-Carboxyalkyl, Carbamoyl oder $C_2$-$C_{16}$-Carbamoylalkyl oder unsubstituiertes oder durch Halogen, Nitro, Hydroxy, oder Carboxy substituiertes Phenyl oder Benzyl bedeuten oder $R^1$ und $R^2$ zusammen eine direkte Bindung bedeuten,
$R^4$ eine unsubstituierte oder durch $C_1$-$C_{12}$-Alkyl, $C_6$-$C_{16}$-Aryl, $C_7$-$C_{16}$-Aralkyl oder $C_3$-$C_8$-Cycloalkyl substituierte Aminogruppe, eine $C_1$-$C_{12}$-Alkoxygruppe, eine $C_5$-$C_8$-Cycloalkoxygruppe, eine $C_6$-$C_{16}$-Aryloxygruppe oder eine $C_7$-$C_{16}$-Aralkoxygruppe bedeutet oder $R^2$ und $R^4$ zusammen eine Gruppe $-CH_2$-CO-$NR^5$ -bilden, oder $R^3$ und $R^4$ zusammen eine Gruppe -CO-$NR^5$-bilden und
$R^5$ Wasserstoff, $C_1$-$C_{12}$-Alkyl, Cyclohexyl, $C_6$-$C_{16}$-Aryl oder $C_7$-$C_{16}$-Aralkyl bedeutet, durch Reaktion eines Mercaptans der Formel II

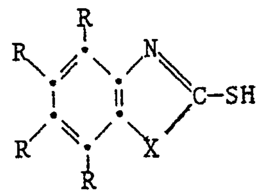

II

mit einem ungesättigten Carbonsäurederivat der Formel III

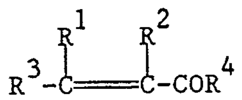

III

in stark saurem Medium.

2. Verfahren gemäss Anspruch 1, worin das Reaktionsmedium eine wässrige oder organisch-wässrige Lösung einer starken Protonsäure ist.

3. Verfahren gemäss Anspruch 2, worin das Reaktionsmedium 60-90 %ige Schwefelsäure oder 25-38 %ige Salzsäure ist.

4. Verfahren gemäss Anspruch 1, dadurch gekennzeichnet, dass die Reaktion bei einer Temperatur von 0° C bis 100° C ausgeführt wird.

5. Verfahren gemäss Anspruch 1, dadurch gekennzeichnet, dass man ein Mercaptan der Formel II verwendet, worin mindestens zwei der Substituenten R Wasserstoff sind.

6. Verfahren gemäss Anspruch 5, worin ein R Wasserstoff, $C_1$-$C_4$-Alkyl, $C_1$-$C_4$-Alkoxy, Halogen, -COOH oder eine Aminogruppe ist und die anderen drei R Wasserstoff sind.

7. Verfahren gemäss Anspruch 1, dadurch gekennzeichnet, dass man eine Verbindung der Formel II verwendet, worin X Schwefel ist.

8. Verfahren gemäss Anspruch 1, dadurch gekennzeichnet, dass man ein Carbcnsäurederivat der Formel III verwendet, worin $R^1$ , $R^2$ und $R^3$ unabhängig voneinander Wasserstoff, $C_1$-$C_8$-Alkyl, Carboxyl, $C_2$-$C_8$-Carbcxyalkyl, Carbamoyl, $C_2$-$C_{16}$-Carbamoylalkyl oder Phenyl bedeuten,
$R^4$ eine Aminogruppe mit bis zu 20 C-Atomen, $C_1$-$C_{12}$-Alkoxy, $C_5$-$C_8$-Cycloalkoxy, $C_6$-$C_{16}$-Aryloxy oder $C_7$-$C_{16}$-Aralkoxy bedeutet oder $R^2$ und $R^4$ zusammen $-CH_2$-CO-$NR^5$- sind oder $R^3$ und $R^4$ zusammen -CO-$NR^5$- sind und
$R^5$ Wasserstoff, $C_1$-$C_{12}$-Alkyl, $C_5$-$C_8$-Cycloalkyl, $C_6$-$C_{16}$-Aryl oder $C_7$-$C_{16}$-Aralkyl bedeutet.

9. Verfahren gemäss Anspruch 1, dadurch gekennzeichnet, dass man ein Carbonsäurederivat der Formel III verwendet, worin mindestens zwei der Substituenten $R^1$, $R^2$ und $R^3$ Wasserstoff sind.

10. Verfahren gemäss Anspruch 1, dadurch gekennzeichnet, dass man ein Carbonsäurederviat der Formel III verwendet, worin $R^3$ Carboxyl oder Carbamoyl ist oder worin $R^2$ Carboxymethyl oder Carbamoylmethyl ist.

11. Verfahren gemäss Anspruch 10, dadurch gekennzeichnet, dass man eine Verbindung der Formel II in stark saurem Medium mit einem Derivat der Maleinsäure oder der Itaconsäure umsetzt.

12. Verfahren gemäss Anspruch 8, dadurch gekennzeichnet, dass man eine Verbindung der Formel III verwendet, worin $R^4$ $C_1$-$C_6$-Alkoxy, Cyclohexyloxy, Cyclopentyloxy, Phenyloxy, Benzyloxy, $-NH_2$ oder $-NR^6R^7$ bedeutet, worin $R^6$ $C_1$-$C_4$-Alkyl ist und $R^7$ Wasserstoff oder $C_1$-$C_4$- Alkyl ist.

**13.** Verfahren gemäss Anspruch 8, dadurch gekennzeichnet, dass man eine Verbindung der Formel III verwendet, worin $R^2$ und $R^4$ zusammen eine Gruppe $-CH_2-CO-NR^5-$ bilden, oder $R^3$ und $R^4$ zusammen eine Gruppe $-CO-NR^5-$ bilden, und $R^5$ Wasserstoff, $C_1$-$C_4$-Alkyl, Cyclohexyl, Phenyl oder Benzyl ist.

## Claims

**1.** A process for the preparation of a compound of the formula I

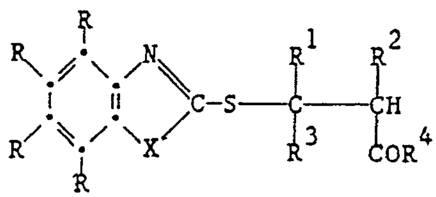

I

in which X is sulfur or NH, each R independently of the others is hydrogen, $C_1$-$C_{12}$alkyl, $C_1$-$C_4$haloalkyl, $C_1$-$C_4$alkoxy, $C_1$-$C_4$alkylthio, $C_1$-$C_{12}$alkylsulfonyl, phenyl, $C_7$-$C_{12}$alkylphenyl, $C_7$-$C_{12}$phenylalkyl, $C_5$-$C_8$cycloalkyl, halogen, $-NO_2$, -CN, -COOH, -COO-$C_1$-$C_4$alkyl, -OH or an amino or carbamoyl group, and $R^1$, $R^2$ and $R^3$ independently of one another are hydrogen, $C_1$-$C_{12}$alkyl, $C_1$-$C_4$haloalkyl, $C_1$-$C_4$hydroxyalkyl, $C_2$-$C_{10}$alkoxyalkyl, carboxyl or $C_2$-$C_{12}$carboxyalkyl, carbamoyl or $C_2$-$C_{16}$carbamoylalkyl, or benzyl or phenyl which is unsubstituted or substituted by halogen, nitro, hydroxyl or carboxyl, or $R^1$ and $R^2$ together are a direct bond, $R^4$ is an amino group which is unsubstituted or substituted by $C_1$-$C_{12}$alkyl, $C_6$-$C_{16}$aryl, $C_7$-$C_{16}$aralkyl or $C_3$-$C_8$cycloalkyl, a $C_1$-$C_{12}$alkoxy group, a $C_5$-$C_8$cycloalkoxy group, a $C_6$-$C_{16}$aryloxy group or a $C_7$-$C_{16}$aralkoxy group, or $R^2$ and $R^4$ together form a group $-CH_2-CO-NR^5-$, or $R^3$ and $R^4$ together form a group $-CO-NR^5-$ and $R^5$ is hydrogen, $C_1$-$C_{12}$alkyl, cyclohexyl, $C_6$-$C_{16}$aryl or $C_7$-$C_{16}$aralkyl, by reaction of a mercaptan of the formula II

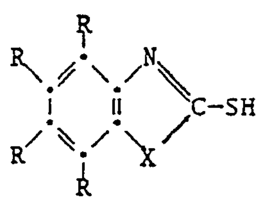

II

with an unsaturated carboxylic acid derivative of the formula III

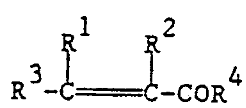

III

in a strongly acid medium.

**2.** A process according to claim 1, wherein the reaction medium is an aqueous or organic-aqueous solution of a strong protonic acid.

**3.** A process according to claim 2, wherein the reaction medium is 60-90% sulfuric acid or 25-38% hydrochloric acid.

**4.** A process according to claim 1, wherein the reaction is carried out at a temperature of 0 °C to 100 °C.

**5.** A process according to claim 1, wherein a mercaptan of the formula II is used in which at least two of the substituents R are hydrogen.

**6.** A process according to claim 5, wherein one R is hydrogen, $C_1$-$C_4$alkyl, $C_1$-$C_4$alkoxy, halogen, -COOH or an amino group and the other three R are hydrogen.

**7.** A process according to claim 1, wherein a compound of the formula II is used in which X is sulfur.

**8.** A process according to claim 1, wherein a carboxylic acid derivative of the formula III is used in which $R^1$, $R^2$ and $R^3$ independently of one another are hydrogen, $C_1$-$C_8$alkyl, carboxyl, $C_2$-$C_8$carboxyalkyl, carbamoyl or $C_2$-$C_{16}$carbamoylalkyl or phenyl, $R^4$ is an amino group having up to 20 C atoms, $C_1$-$C_{12}$alkoxy, $C_5$-$C_8$cycloalkoxy, $C_6$-$C_{16}$aryloxy or $C_7$-$C_{16}$aralkoxy, or $R^2$ and $R^4$ together are $-CH_2-CO-NR^5-$ or $R^3$ and $R^4$ together are $-CO-NR^5-$, and $R^5$ is hydrogen, $C_1$-$C_{12}$alkyl, $C_5$-$C_8$cycloalkyl, $C_6$-$C_{16}$aryl or $C_7$-$C_{16}$aralkyl.

**9.** A process according to claim 1, wherein a carboxylic acid derivative of the formula III is used in which at least two of the substituents $R^1$, $R^2$ and $R^3$ are hydrogen.

**10.** A process according to claim 1, wherein a carboxylic acid derivative of the formula III is used in which $R^3$ is carboxyl or carbamoyl or in which $R^2$ is carboxymethyl or carbamoylmethyl.

**11.** A process according to claim 10, wherein a compound of the formula II is reacted with a maleic acid derivative or an itaconic acid derivative in a strongly acid medium.

**12.** A process according to claim 8, wherein a compound of the formula III is used in which $R^4$ is $C_1$-$C_6$alkoxy, cyclohexyloxy, cyclopentyloxy, phenyloxy, benzyloxy, $-NH_2$ or $-NR^6R^7$, in which $R^6$ is $C_1$-$C_4$alkyl and $R^7$ is hydrogen or $C_1$-$C_4$alkyl.

**13.** A process according to claim 8, wherein a compound of the formula III is used in which $R^2$ and $R^4$ together form a group $-CH_2-CO-NR^5-$, or $R^3$ and $R^4$ together form a group $-CO-NR^5-$, and $R^5$ is hydrogen, $C_1$-$C_4$alkyl, cyclohexyl, phenyl or benzyl.

## Revendications

**1.** Procédé pour préparer des composés répondant à la formule I :

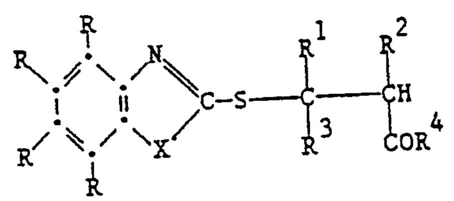

I,

dans laquelle :
X représente le soufre ou NH,
les R représentent chacun, indépendamment les uns des autres, l'hydrogène, un alkyle en $C_1$-$C_{12}$, un halogénoalkyle en $C_1$-$C_4$, un alcoxy en $C_1$-$C_4$, un alkylthio en $C_1$-$C_4$, un alkylsulfonyle en $C_1$-$C_{12}$, un phényle, un alkylphényle en $C_7$-$C_{12}$, un phénylalkyle en $C_7$-$C_{12}$, un cycloalkyle en $C_5$-$C_8$, un halogène, $-NO_2$, -CN, -COOH, un alcoxycarbonyle à alkyle en $C_1$-$C_4$, -OH, un amino ou un carbamoyle,
$R^1$, $R^2$ et $R^3$ représentent chacun, indépendamment les uns des autres, l'hydrogène, un alkyle en $C_1$-$C_{12}$, un halogénoalkyle en $C_1$-$C_4$, un hydroxyalkyle en $C_1$-$C_4$, un alcoxyalkyle en $C_2$-$C_{10}$, un carboxy, un carboxyalkyle en $C_1$-$C_{12}$, un carbamoyle ou un carbamoylalkyle en $C_2$-$C_{16}$, ou un radical phényle ou benzyle non substitué ou porteur d'un halogène, d'un nitro, d'un hydroxy ou d'un carboxy, ou $R^1$ et $R^2$ forment ensemble une liaison directe,
$R^4$ représente un amino non substitué ou porteur d'un alkyle en $C_1$-$C_{12}$, d'un aryle en $C_6$-$C_{16}$, d'un aralkyle en $C_7$-$C_{16}$ ou d'un cycloalkyle en $C_3$-$C_8$, un alcoxy en $C_1$-$C_{12}$, un cycloalcoxy en $C_5$-$C_8$, un aryloxy en $C_6$-$C_{16}$ ou un aralcoxy en $C_7$-$C_{16}$, ou $R_2$ et $R_4$ forment ensemble un radical $-CH_2-CO-NR^5$, ou $R^3$ et $R^4$ forment ensemble un radical $-CO-NR^5$, et
$R^5$ représente l'hydrogène, un alkyle en $C_1$-$C_{12}$, un cyclohexyle, un aryle en $C_6$-$C_{16}$ ou un aralkyle en $C_7$-$C_{16}$,
par réaction d'un thiol de formule II :

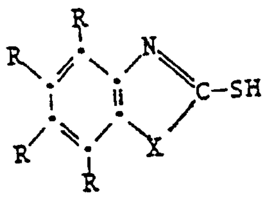

II,

avec un dérivé d'acide carboxylique insaturé de formule III:

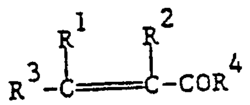

III

dans un milieu très acide.

2. Procédé selon la revendication 1 caractérisé en ce que le milieu réactionnel est une solution aqueuse ou organique-aqueuse d'un acide protonique fort.

3. Procédé selon la revendication 2 caractérisé en ce que le milieu réactionnel est un acide sulfurique à 60-90% ou un acide chlorhydrique à 25-38%.

4. Procédé selon la revendication 1 caractérisé en ce que la réaction est effectuée à une température de 0 à 100° C.

5. Procédé selon la revendication 1 caractérisé en ce qu'on utilise un thiol de formule II dans lequel au moins deux des substituants R représentent chacun l'hydrogène.

6. Procédé selon la revendication 5 caractérisé en ce que R représente l'hydrogène, un alkyle en $C_1$-$C_4$, un alcoxy en $C_1$-$C_4$, un halogène, un -COOH ou un amino, et les trois autres R représentent chacun l'hydrogène.

7. Procédé selon la revendication 1 caractérisé en ce qu'on utilise un composé de formule II dans lequel X représente le soufre.

8. Procédé selon la revendication 1 caractérisé en ce qu'on utilise un dérivé d'acide carboxylique de formule III dans lequel $R^1$, $R^2$ et $R^3$ représentent chacun, indépendamment les uns des autres, l'hydrogène, un alkyle en $C_1$-$C_8$, un carboxy, un carboxy-alkyle en $C_2$-$C_8$, un carbamoyle, un carbamoyl-alkyle en $C_2$-$C_{16}$ ou un phényle, $R^4$ représente un radical amino contenant au plus 20 atomes de carbone, un alcoxy en $C_1$-$C_{12}$, un cycloalcoxy en $C_5$-$C_8$, un aryloxy en $C_6$-$C_{16}$ ou un aralcoxy en $C_7$-$C_{16}$, ou $R^2$ et $R^4$ forment ensemble un radical $-CH_2-CO-NR^5$, ou $R^3$ et $R^4$ forment ensemble un radical $-CO-NR^5$, et R5 représente l'hydrogène, un alkyle en $C_1$-$C_{12}$, un cycloalkyle en $C_5$-$C_8$, un aryle en $C_6$-$C_{16}$ ou un aralkyle en $C_7$-$C_{16}$.

9. Procédé selon la revendication 1 caractérisé en ce qu'on utilise un dérivé d'acide carboxylique de formule III dans lequel au moins deux des symboles $R^1$, $R^2$ et $R^3$ représentent chacun l'hydrogène.

10. Procédé selon la revendication 1 caractérisé en ce qu'on utilisé un dérivé d'acide carboxylique de formule III dans lequel $R^3$ représente un carboxy ou un carbamoyle, ou dans lequel $R^2$ représente un carboxyméthyle ou un carbamoylméthyle.

11. Procédé selon la revendication 10 caractérisé en ce qu'on fait réagir un composé de formule II dans un milieu très acide avec un dérivé de l'acide maléique ou de l'acide itaconique.

12. Procédé selon la revendication 8 caractérisé en ce qu'on utilise un composé de formule III dans lequel $R^4$ représente un alcoxy en $C_1$-$C_6$, un cyclohexyloxy, un cyclopentyloxy, un phényloxy, un benzyloxy,

un radical -NH2 ou un radical $-NR^6R^7$ dans lequel $R^6$ représente un alkyle en $C_1$-$C_4$ et $R^7$ l'hydrogène ou un alkyle en $C_1$-$C_4$.

**13.** Procédé selon la revendication 8 caractérisé en ce qu'on utilise un composé de formule III dans lequel $R^2$ et $R^4$ forment ensemble un radical $-CH_2-CO-NR^5$, ou $R^3$ et $R^4$ forment ensemble un radical $-CO-NR^5$, et $R^5$ représente l'hydrogène, un alkyle en $C_1$-$C_4$, un cyclohexyle, un phényle ou un benzyle.